# EUROPEAN PATENT SPECIFICATION

(11) **EP 0 830 083 B1**
(45) Date of publication and mention of the grant of the patent: **07.03.2007**
(21) Application number: 96916659.4
(22) Date of filing: 24.05.1996
(51) Int. Cl.: A61B 17/00, A61F 2/16

(54) **INJECTION SYSTEM FOR A DEFORMABLE INTRAOCULAR LENS**
EINSPRITZSYSTEM FÜR EINE DEFORMIERBARE INTRAOKULARE LINSE
SYSTEME D'INJECTION POUR UNE LENTILLE INTRA-OCULAIRE DEFORMABLE

(30) Priority: 24.05.1995 US 449103
(43) Date of publication of application: 25.03.1998
(73) Proprietor: Staar Surgical Company, Monrovia, CA 91016 (US)
(72) Inventor: Chambers, Thomas J., Upland, CA 91784 (US)
(74) Representative: Cawdell, Karen Teresa
(86) International application number: PCT/US1996/007750
(87) International publication number: WO 1996/037152

(56) References cited:
- EP-A- 0 363 213
- WO-A-96/03924
- US-A- 4 597 753
- US-A- 4 681 102
- US-A- 4 765 329
- US-A- 5 304 182
- PATENT ABSTRACTS OF JAPAN vol. 017, no. 446 (C-1098), 17 August 1993 (1993-08-17) & JP 05 103809 A (CANON STAR KK), 27 April 1993 (1993-04-27)

## Description

### Field of the Invention

This invention is directed to a deformable intraocular lens injecting system for surgical implantation of a deformable intraocular lens in the eye. The deformable intraocular lens injecting system preferably comprises a lens injecting device and a lens cartridge.

### Background of the Invention

Presently, deformable intraocular lens are being surgically implanted by various techniques. The majority of these surgical procedures in the United States and abroad involve the use of either 1) surgical forceps; or 2) "shooter" devices for mechanically deforming (e.g., folding, compressing, condensing, rolling, etc.) and manipulating the deformable intraocular lens in a manner to allow insertion through a small incision (2.5 to 3.0 mm) in the eye.

STAAR Surgical Company of Monrovia, California, the inventors and originators of the deformable intraocular lens with Dr. Mozzocco, have also been the innovators and suppliers of "shooter" devices to the industry. These "shooter" devices have been widely accepted and currently used by surgeons specializing in the implantation of deformable intraocular lenses.

STAAR Surgical Company informally estimates that approximately fifty (50) percent of all surgical procedures involving the implantation of deformable intraocular lens occur with the use of "shooter" devices.

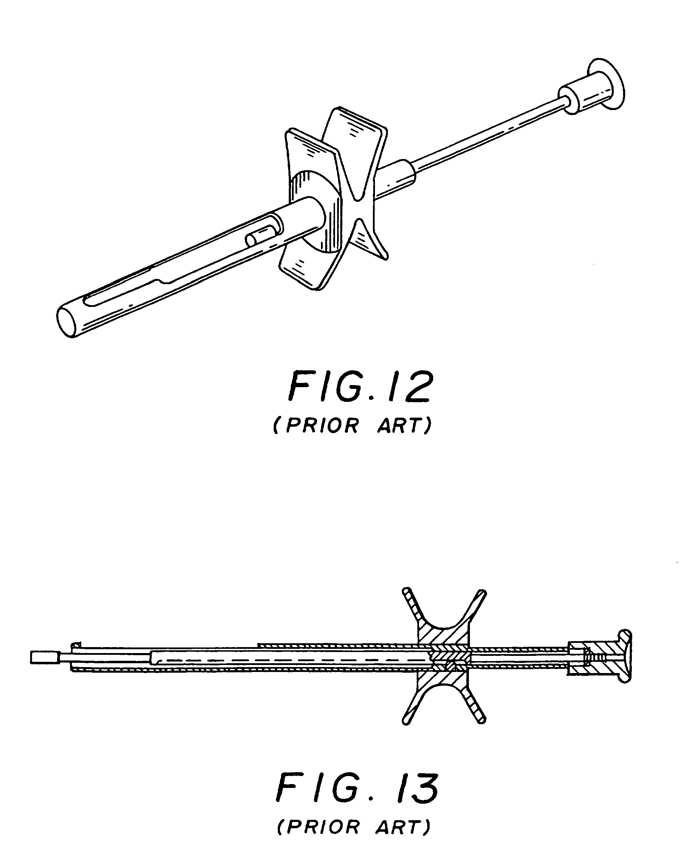

The original "shooter" devices proposed and designed by Dr. Mozzocco were configured to directly receive a loose unprotected deformable intraocular lens. In such devices, the fully exposed deformable intraocular lens could be potentially damaged while loading the "shooter" device with the deformable intraocular lens. In further developing "shooter" type devices, STAAR Surgical Company made the first "shooter" system comprising an injecting device and a separate one-piece foldable lens cartridge having a lens holding portion connected to a nozzle portion. This development resulted in a "shooter" device that became known in the industry as the "STAAR Shooter". The "STAAR Shooter" became available around 1986, and was supplied on an experimental use basis to surgeons participating in clinical studies seeking approval of implantation of deformable intraocular lens in the human eye by the Food and Drug Administration (FDA), which approval occurred in 1991. An example of a prior art shooter device of STAAR Surgical Company is shown in Figures 12 and 13.

The prior art shooter device shown in Figures 12 and 13 includes a freely slidable plunger that can be advanced by simply pushing on the one end of the plunger. The slidable plunger includes a tip for contacting with the deformable intraocular lens loaded in a foldable type lens cartridge and forcing the deformable intraocular lens through a nozzle portion of the lens cartridge inserted through a small incision in the eye.

The standard method of use of the prior art shooter device comprises the steps of:
1) loading a deformable intraocular lens into an foldable type lens cartridge opened for receiving the deformable intraocular lens in a flat configuration;
2) closing the foldable type lens cartridge which causes the deformable intraocular lens to fold inside the passageway through the lens cartridge;
3) inserting the loaded lens cartridge into the "STAAR Shooter" device;
4) inserting the tip of the nozzle portion of the lens cartridge through the incision into the eye;
5) advancing the plunger by pushing on the end of the plunger with the thumb while gripping the finger grip connected to the barrel with index finger above and middle finger below so that the tip of the plunger enters into the end of the passageway through the lens cartridge, and then contacts with a trailing end of the deformable intraocular lens; and
6) further advancing the plunger by further pushing on the end of the plunger so that the plunger tip forces the deformable intraocular lens through the passageway in the lens cartridge and out through a tip of the nozzle portion extending through the incision in the eye, thus, allowing the unconstrained deformable intraocular lens to open up (i.e. unfold or decompress) inside the eye.

The manner in which the deformable intraocular lens is released from the tip of the nozzle portion inside the eye can be an important factor with respect to the degree of success of the surgical procedure, and risk of harm or damage to eye tissue during the surgical procedure. In order to increase the degree of success of the surgical procedure and minimize the risk to the patient, it is highly desirable that the deformable intraocular lens is released in a controlled manner, since uncontrolled release of the deformable intraocular lens could cause eye damage. Specifically, due to the resilient nature of the plastic material of the deformable intraocular lens and the plastic material of the nozzle portion of the lens cartridge, the deformable intraocular lens has a tendency to shoot out of the end of the tip of the nozzle portion when uncontrollably released. This phenomenon occurs due to the highly compressed or constrained (i.e. "stressed") configuration of the deformed intraocular lens attempting to expand inside the nozzle portion having resilient walls. When a certain portion of the deformable intraocular lens is extruded or projected a certain critical distance outside the opening of the tip of the nozzle portion, the "stressed" lens will tend to suddenly release and shoot out of the tip of the nozzle portion into the interior of the eye. This phenomenon can result in torn capsular bags and/or damage to tissue in the anterior and posterior chambers in some cases.

In order to control the release of the intraocular lens, various known modifications to the shooters and lens cartridges have been made, and insertion techniques have also been modified. For example, the tip of the nozzle portion can be provided with slits that control the opening and alleviate the expansion force of the folded or compressed intraocular lens exiting the tip of the nozzle portion. Further, the plunger in some shooters is not freely slidable, but instead provided with threaded means for advancing the movement of the plunger. For example, STAAR Surgical Company currently provides a "shooter" under the Model No. MSI-T having a plunger with threaded advancement means allowing for highly controlled fine advancement of the plunger, which provides some controlled release of the deformable intraocular lens.

However, this type of "shooter" having threaded advancing means for controlling the movement of the plunger is not considered desirable by some high volume and high skilled surgeons performing multiple surgical procedures one after another, since any means for restricting the movement of the plunger impedes the surgeon increasing the time for each procedure and potentially limits the manoeuverability of the "shooter".

WO96/03924 discloses a device for inserting a flexible intraocular lens into an eye comprising a tubular member and a plunger. The tubular member includes a staging area, a lumen, and an open distal end. The staging area supports the lens in an unstressed state prior to engagement by the plunger. In the unstressed state, the optic of the lens is suspended in a pocket to avoid any substantial contact with interior portions of the tubular member. The plunger includes a slot in its distal tip for receiving and gripping the lens. With this construction, the lens can be inserted into the eye in one continuous motion. Further, the plunger holds the lens when the lens is moved out of the tubular member.

EP0363213 discloses another intraocular lens insertion instrument including a holder for holding a folded optic and a plunger having an enlarged head for forcing the folded optic from the holder while a haptic of the lens is protected from damage in a space within the holder created by a portion of the piston's barrel having a reduced cross-section.

US4765329 discloses yet another intraocular lens insertion instrument comprising an insertion tube with a tip of reduced diameter which is inserted into the eye through a small incision; and a probe which is inserted into the tube to force an intraocular lens contained in the tube through the tip of the tube and into the eye. The handle of the probe is shaped so that its linear movement into the tube is stopped at a predetermined linear position of the probe in the tube, so as to limit the displacement of the probe into the tip of the tube.

A further intraocular lens insertion instrument is disclosed in JP05103809. This device has an elastic optical part which is inserted in the insertion cylinder of a holder; the holder being attached to the main body of the device. A shaft disposed in the main body of the device is advanced by a drive mechanism to push out the intraocular lens.

Yet a further intraocular lens insertion instrument is disclosed in US 4681 102, which discloses the preamble of claim 1. This device comprises a cylindrical load chamber having a pair of flanges, the load chamber being folded around the intraocular lens, so that the lens is folded or rolled along its length. The load chamber is fitted into an injector portion, which has a slot which is keyed to the flanges of the load chamber. A plunger is inserted into the injector portion to push the intra-ocular lens through the lumen and into the eye.

Thus, it is highly desirable to improve both the devices and methods of implanting the deformable intraocular lens in the eye to provide controlled release of the deformable intraocular lens into the eye, and for decreasing the time of the surgical procedure. Also, the ergonomic 'feel' for the surgeon using the 'shooter' devices can be enhanced by 'shooters' according to the present invention tending to increase surgical efficiency and decrease the risk to the patient.

An object of the present invention is to provide a deformable intraocular lens injecting system comprising a lens injecting device having a slidable plunger with a spring and a damper to control the movement of the plunger and in effect control the release of the deformable intraocular lens into the eye.

The present invention is directed to apparatus for controlling the release of the advancing folded or condensed deformable intraocular lens by applying a force in a direction opposite to the direction of advancement of the deformable lens when being inserted into the eye. The force can be a constant or variable force, and the force can be selectively applied. These different types of forces can be applied mechanically, fluidly, pneumatically, magnetically, electrically, electromagnetically, combinations of forces, etc. However, mechanically means of applying the force by a spring and/or damper arrangement(s) provide for simple, inexpensive and reliable designs, and currently appear to be the most promising for future developments.

Various configurations of a spring or combination of springs can be applied in the present invention. For example, a coiled spring, cantilever spring, or spring arrangement utilizing the resilient property of the spring's structural configuration and/or the elastic property of the material forming the spring (e.g. plastic resin tubing, longitudinal slits in sides of harder plastic tubing providing a plurality of plate-like springs connected together at ends, plastic or rubber grommets or diaphragms).

The lens injecting device according to the present invention is preferably configured to apply an opposite force to the advancing plunger when the deformable intraocular lens is exiting the nozzle of the lens injecting device to provide controlled release of the deformable intraocular lens into the eye. In a preferred embodiment, the opposite force is applied to the advancing plunger only near the end of its stroke when the deformable intraocular lens is about to exit the tip of the nozzle. This arrangement allows the plunger to freely slide unimpeded most of the distance of its stroke yet provide sufficient back pressure at the end of its stroke to prevent sudden plunger advancement as the intraocular lens exits the nozzle.

### Brief Description of the Drawings

Fig. 1 is a side elevational view of a preferred embodiment of the lens injecting device according to the present invention.
Fig. 2 is a top planar view of the lens injecting device shown in Fig. 1.
Fig. 3 is a side elevational view of the slidably disposed plunger dissembled from the lens injecting device shown in Fig. 1 to show its detailed structural configuration.
Fig. 4 is a detailed cross-sectional view of an alternative embodiment of the thumb grip having a cylindrical recess to accommodate a fastener having a threaded bore.
Fig. 5 is a detailed broken away cross-sectional view showing the manner in which the sleeve stop mounted on the slidable plunger engages with an end face of the cylindrical barrel to engage the coiled spring causing a spring force opposite in direction to the advancing slidable plunger.
Fig. 6 is a detailed cross-sectional view as indicated in Fig. 1 of the cylindrical barrel and finger grip.
Fig. 7 is a partial broken away view of an alternative spring made of resilient material, e.g. plastic, mounted on the slidable plunger.
Fig. 8 is a detailed longitudinal cross-sectional view of an alternative sleeve stop having a frictional lining to function as a damper to provide resistance of movement of the advancing slidable plunger.
Fig. 9 is a diagrammatic view of a slidable plunger provided with a spring for applying force in an opposite direction of advancement of the slidable plunger.
Fig. 10 is a diagrammatic view of a slidable plunger provided with a damper to function as a damper to provide resistance of movement of the advancing slidable plunger.
Fig. 11 is a diagrammatic view of a slidable plunger provided with both a spring and damper.
Fig. 12 is a perspective view of a prior art embodiment of a shooter device of STAAR Surgical Company of Monrovia, California.
Fig. 13 is a longitudinal cross-sectional view of the prior art shooter device shown in Fig. 13.

### Detailed Description of Preferred Embodiments

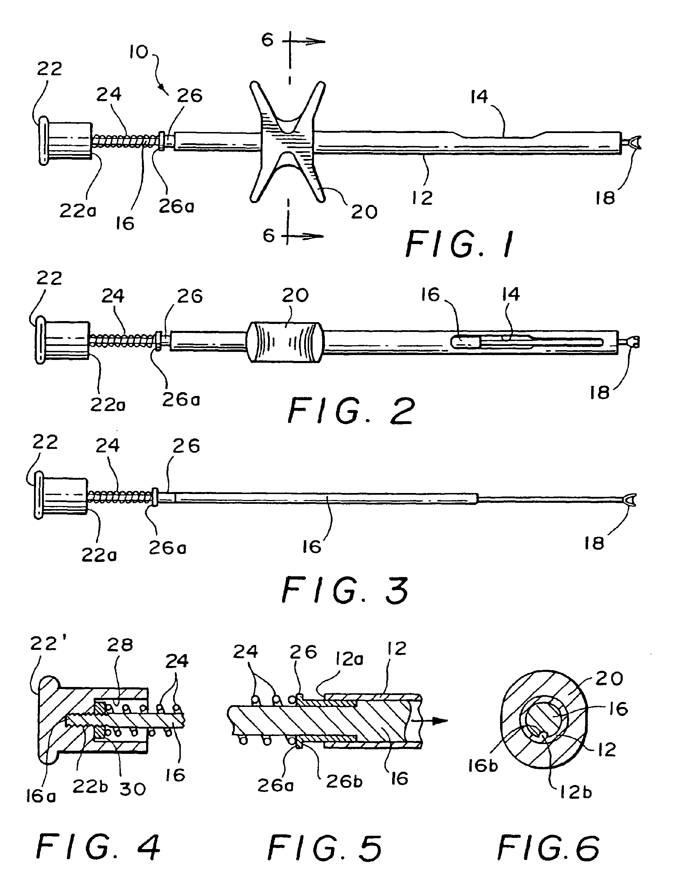

A preferred embodiment of a lens injecting device 10 as shown in Figures 1 and 2. This lens injecting device is configured for receiving a lens cartridge having a lens holding portion connected to a nozzle portion, and disclosed in the parent applications referred to in the section under related applications.

The lens injecting device 10 comprises a cylindrical barrel 12 having a lens cartridge receiver 14, and a slidable plunger 16 having a plunger tip 18. Further, the cylindrical barrel 12 is provided with a finger grip 20 and the slidable plunger 16 is provided with a thumb grip 22 for manipulating the lens injecting device 10.

In the preferred embodiment shown in Figures 1 and 2, a spring 24 is provided to create a force between the cylindrical barrel 12 and slidable plunger 16. Specifically, the coil type spring 24 is wrapped around a portion of the plunger 16, as indicated on the left-hand side of Figure 3. Further, the coil type spring 24 extends between the thumb grip 22 and the sleeve stop 26. The face of fastener nut 30 and the end face 26a act as end stops for the spring 24 causing the spring 24 to functionally engage with the slidable plunger 16 when the slidable plunger 16 is moved in a right-ward direction sufficiently to engage an opposite end face 26b of the sleeve stop 26 with the end face 12a of the cylindrical barrel 12, as shown in detail in Figure 5.

The thumb grip 22 is connected to one end of the slidable plunger 16. For example, the one end of the slidable plunger 16 can be provided with a threaded end portion 16a to be threaded into a threaded bore 22b of the thumb grip 22. In this embodiment of the thumb grip 22', the thumb grip 22' is provided with a cylindrical recess 28 for accommodating a fastener nut 30 threaded onto the end of threaded end portion 16a of the slidable plunger 16. This configuration allows the coil spring 24 to be positioned on the end portion of the slidable plunger 16 and locked in place by the fastener nut 30 prior to the threaded end portion 16a of the slidable plunger 16 being threaded into the threaded bore 22b of the thumb grip 22. This configuration facilitates assembly of the lens injecting device, and more securely restrains the coil spring 24 on the one end of the plunger 16.

The plunger 16 is provided with a lower groove 16b for receiving a inwardly extending protrusion 12b of the cylindrical barrel 12 to provide a locking key way arrangement to prevent relative rotation of the slidable plunger 16 within the cylindrical barrel 12 (i.e., maintains a fixed orientation of the slidable plunger 16 to prevent rotation within the cylindrical barrel 12). Further, the finger grip 20 is securely connected to the cylindrical barrel 12 to prevent any relative movement there between. For example, the finger grip 20 is provided with a through bore that interference fits with the outer surface of a portion of the cylindrical barrel 12.

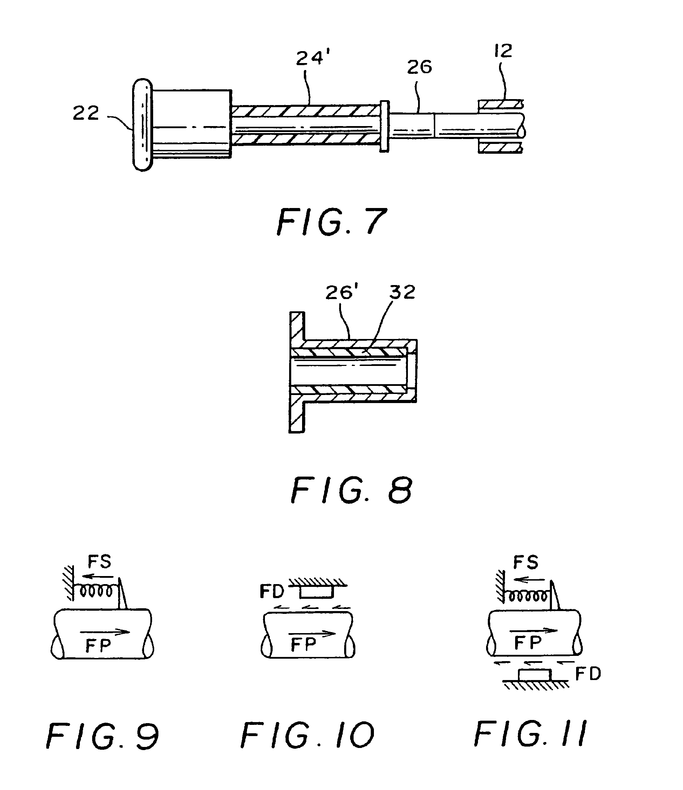

In an alternative embodiment as shown in Figure 7, the coil spring 24 is replaced with a section of resilient rubber or plastic tubing 24' performing the function of a spring due to the resilient nature of the material making up the section of tubing. As a further alternative, as shown in Figure 8, a sleeve stop 26' having a frictional lining 32 (e.g., synthetic plastic, ceramic, metal, glass, etc.) provides somewhat of a friction fit with an outer surface of the plunger to function as a damper resisting the forward movement of the slidable plunger to control release of the deformable intraocular lens from the tip of the nozzle portion of the lens cartridge.

The examples of springs and dampers discussed above are only illustrative examples embodying the concepts of the present invention, however, other types of springs (e.g., cantilever type) and/or dampers (e.g., viscous fluid medium between plunger and cylindrical barrel) can be provided as alternatives to the preferred embodiment shown.

Figures 9 through 11 schematically illustrate applying a spring force, applying a damping resistance force, and combining both spring force and damping resistance force, respectively, for controlling the forward movement of the slidable plunger and for controlling the release of the deformable intraocular lens from the nozzle of the injecting device.

## Claims

1. A deformable intraocular lens injecting device (10) for inserting a deformable intraocular lens through a small incision in the eye, comprising a sleeve (12) having a first end having an opening and a second end having an opening and a passage way formed therebetween, the sleeve having a lens cartridge receiver (14) disposed in a side of the sleeve, the lens cartridge receiver (14) for accommodating a lens cartridge containing the deformable intraocular lens, a plunger (16) slidably received within the passageway of the sleeve (12), the plunger (16) being inserted through the first opening of the sleeve (12), the plunger (16) having a plunger tip (18) fur contacting the deformable intraocular lens at one end and a grip (22) for actuating said plunger (16) at an opposite end thereof, **characterized by** a spring (24) associated with the plunger (16) for providing a respective spring force against the plunger when the plunger (16) is forced through the sleeve (12) and out the second opening of the sleeve,
and a damper (26') associated with the plunger (16) for providing resistance against movement of the plunger (16) when the plunger is forced through the sleeve (12).

2. The injecting device of claim 1, **characterized in that** the lens cartridge receiver (14) is a slot disposed in a side of the sleeve (12) adjacent the second opening.

3. The injecting device of claim 1, **characterized in that** the spring (24) or damper (26') is arranged to constantly engage the plunger (16).

4. The injecting device of claim 1, **characterized in that** the spring (24) or damper (26') is arranged to engage with the plunger (16) when the plunger is reaching an end of a full length of its delivery stroke.

5. The injecting device of claims 3 and 4, wherein said spring or damper engages the plunger (16) when the plunger tip (18) is located in the passageway.

6. The injecting device of claim of any of claims 1 to 5, **characterized in that** the spring (24) provides sufficient spring force on the plunger (16) to cause the plunger to rebound to some extent after the plunger reaches its full length delivery stroke.

7. The injecting device of claim 6, wherein the spring (24) provides a sufficient spring force to fully withdraw the plunger tip (18) back inside the passageway.

8. The injecting device of any of claims 1 to 7, further **characterized by** a lens cartridge for containing the deformable intraocular lens, the lens cartridge having a nozzle portion connected to a lens holding portion.

9. The injecting device of claim 8, **characterized in that** the nozzle portion has one or more frangible walls for controlling the release of the deformable intraocular lens into the eye.

## Patentansprüche

1. injektionsvorrichtung (10) für eine verformbare, intraokulare Linse zum Einsetzen einer verformbaren, intraokularen Linse durch einen kleinen Einschnitt in das Auge, umfassend eine Hülse (12), welche ein erstes Ende mit einer Öffnung und ein zweites Ende mit einer Öffnung und einen Durchgangsweg aufweist, der dazwischen ausgebildet ist, wobei die Hülse eine Linsenpatronenaufnahme (14) hat, die in einer Seite der Hülse angeordnet ist, die Linsenpatronenaufnahme (14) zum Aufnehmen einer Linsenpatrone, welche die verformbare, intraokulare Linse beinhaltet, ausgebildet ist, einen Kolben (16), der gleitend innerhalb des Durchgangsweg der Hülse (12) aufgenommen ist, wobei der Kolben (16) durch die erste Öffnung der Hülse (12) eingeführt wird und eine Kolbenspitze (18) zum Kontaktieren der verformbaren, intraokularen Linse an einem Ende und einen Griff (22) zum Betätigen des Kolbens (16) an einem gegenüberliegenden Ende desselben aufweist, und einen Dämpfer (26'), der dem Kolben (16) zugeordnet ist zum Bereitstellen eines Widerstands gegen Bewegung des Kolbens (16), wenn der Kolben durch die Hülse (12) forciert wird, **gekennzeichnet durch** eine Feder (24), welche dem Kolben (16) zugeordnet ist zum Bereitstellen einer entsprechenden Federkraft gegen den Kolben, wenn der Kolben **durch** die Hülse (12) und aus der zweiten Öffnung der Hülse forciert wird.

2. Injektionsvorrichtung nach Anspruch 1, **dadurch gekennzeichnet, dass** die Linsenpatronenaufnahme (14) ein Schlitz ist, der in einer Seite der Hülse (12) benachbart zur zweiten Öffnung angeordnet ist.

3. Injektionsvorrichtung nach Anspruch 1, **dadurch gekennzeichnet, dass** die Feder (24) oder der Dämpfer (26') angeordnet ist, um konstant mit dem Kolben (16) zusammenzuwirken.

4. Injektionsvorrichtung nach Anspruch 1, **dadurch gekennzeichnet, dass** die Feder (24) oder der Dämpfer (26') angeordnet ist, um mit dem Kolben (16) zusammenzuwirken, wenn der Kolben ein Ende einer vollen Länge seines Überführungshubs erreicht.

5. Injektionsvorrichtung nach den Ansprüchen 3 und 4, worin die Feder oder der Dämpfer mit dem Kolben (16) zusammenwirkt, wenn die Kolbenspitze (18) in dem Durchgangsweg angeordnet ist.

6. Injektionsvorrichtung nach einem der Ansprüche 1 bis 5, **dadurch gekennzeichnet, dass** die Feder (24) ausreichend Federkraft auf den Kolben (16) ausübt, um zu bewirken, dass der Kolben zu einem gewissen Ausmaß zurückfedert, nachdem der Kolben seinen vollen Überführungshub erreicht.

7. Injektionsvorrichtung nach Anspruch 6, worin die Feder (24) eine ausreichende Federkraft bereitstellt, um die Kolbenspitze (18) vollständig in den Durchgangsweg zurückzuziehen.

8. Injektionsvorrichtung nach einem der Ansprüche 1 bis 7, weiter **gekennzeichnet durch** eine Linsenpatrone zum Aufnehmen der verformbaren, intraokularen Linse, wobei die Linsenpatrone einen Düsenabschnitt aufweist, der mit einem Linsenhalteabschnitt verbunden ist.

9. Injektionsvorrichtung nach Anspruch 8, **dadurch gekennzeichnet, dass** der Düsenabschnitt eine oder mehrere zerbrechliche Wände zum Steuern der Freigabe der verformbaren, intraokularen Linse in das Auge aufweist.

## Revendications

1. Dispositif d'injection (10) de lentille intra-oculaire déformable pour insérer une lentille intra-oculaire déformable au travers d'une petite incision dans l'oeil, comprenant un manchon (12) possédant une première extrémité ayant une ouverture et une seconde extrémité ayant une ouverture et un passage formé entre celles-ci, le manchon ayant un logement (14) de cartouche de lentille disposé sur un côté du manchon, le logement (14) de cartouche de lentille pour accueillir une cartouche de lentille contenant la lentille intra-oculaire déformable, un piston (16) étant coulissé pour être disposé à l'intérieur du passage du manchon (12), le piston (16) étant inséré au travers de la première ouverture du manchon (12), le piston (16) ayant une extrémité de piston (18) pour être en contact avec la lentille intra-oculaire déformable à une extrémité et une griffe (22) pour actionner ledit piston (16) à l'extrémité opposée de celui-ci, **caractérisé par** un ressort (24) associé au piston (16) afin d'exercer une force du ressort respective contre le piston lorsque le piston (16) est contraint de passer au travers du manchon (12) et est contraint de sortir par la seconde ouverture du manchon, et un amortisseur (26') associé au piston (16) pour fournir une résistance au mouvement du piston (16) lorsque le piston est contraint de passer au travers du manchon (12).

2. Dispositif d'injection selon la revendication 1, **caractérisé en ce que** le logement (14) de cartouche de lentille est une fente disposée sur un côté du manchon (12) adjacent à la seconde ouverture.

3. Dispositif d'injection selon la revendication 1, **caractérisé en ce que** le ressort (24) ou l'amortisseur (26') est disposé de façon à venir en contact constant avec le piston (16).

4. Dispositif d'injection selon la revendication 1, **caractérisé en ce que** le ressort (24) ou l'amortisseur (26') est disposé de façon à venir en contact avec le piston (16) lorsque le piston atteint une extrémité d'une longueur totale de sa course de distribution.

5. Dispositif d'injection selon les revendications 3 et 4, dans lequel ledit ressort ou amortisseur vient en contact avec le piston (16) lorsque l'extrémité du piston (18) est située dans la voie de passage.

6. Dispositif d'injection selon l'une quelconque des revendications 1 à 5, **caractérisé en ce que** le ressort (24) exerce une force de ressort suffisante sur le piston (16) afin de permettre au piston de rebondir dans une certaine mesure après que le piston a atteint sa course de distribution de longueur totale.

7. Dispositif d'injection selon la revendication 6, dans lequel le ressort (24) exerce une force de ressort suffisante pour que l'extrémité du piston (18) se rétracte totalement à l'intérieur de la voie de passage.

8. Dispositif d'injection selon l'une quelconque des revendications 1 à 7, **caractérisé en outre par** une cartouche de lentille pour contenir la lentille intra-oculaire déformable, la cartouche de lentille ayant une partie de buse connectée à une partie de maintien de lentille.

9. Dispositif d'injection selon la revendication 8, **caractérisé en ce que** la partie de buse possède une ou plusieurs parois frangibles pour commander la libération de la lentille intra-oculaire déformable à l'intérieur de l'oeil.
